# EUROPEAN PATENT APPLICATION

(11) **EP 4 550 352 A1**
(43) Date of publication of application: **07.05.2025**
(21) Application number: 23846984.5
(22) Date of filing: 26.07.2023
(51) Int. Cl.: G16H 50/20, G16H 50/30, G16H 50/50, G16H 50/70, G16H 30/20, G16H 80/00, G16H 10/60, A61B 5/346, A61B 5/00

(54) **METHOD, PROGRAM, AND DEVICE FOR PROVIDING ECG INTERPRETATION SERVICE**

(30) Priority: 29.07.2022 KR 20220094607; 25.07.2023 KR 20230096757
(71) Applicant: Medical AI Co., Ltd., Seoul 06180 (KR)
(72) Inventor: KWON, Joonmyoung, Seoul 06180 (KR); LIM, Seonyu, Seoul 06180 (KR)
(74) Representative: Patentanwaltskanzlei Matschnig & Forsthuber OG
(86) International application number: PCT/KR2023/010799
(87) International publication number: WO 2024/025326

(57) **Abstract**

According to one embodiment of the present disclosure, there are disclosed a method, program, and device for providing an electrocardiogram reading service that are performed by a computing device. The method may include: generating first additional information about reading results included in a reading text by analyzing the reading text generated using a first machine learning model pre-trained to diagnose a disease based on an electrocardiogram; generating information related to the first machine learning model, used to generate the reading text, as second additional information; and providing at least one of the first additional information and the second additional information through a user interface.

## Description

### Technical Field

The present disclosure relates to data processing technology in the medical field, and more particularly, to a method capable of processing and additionally providing information that can supplement the results of electrocardiogram reading using artificial intelligence.

### Background Art

Conventional automatic electrocardiogram reading simply tells us the disease that may be present based on an electrocardiogram. However, information about a disease that may be present alone is not helpful to a doctor who checks the results of electrocardiogram reading. Since doctors who use an electrocardiogram reading service (i.e., doctors who request an electrocardiogram reading service) are doctors who lack specialized knowledge in electrocardiogram reading, they do not know a test or treatment to prescribe in order to treat a disease even when they are aware of reading results indicating that the disease is suspected in an electrocardiogram. Accordingly, it is necessary to tell such a requester (such a non-specialist) not only that there is disease A, but also that a specific additional test needs to be performed and a specific prescription needs to be made.

In addition, in the conventional automatic electrocardiogram reading, the basis for reading results is not described, so that there is an aspect that makes it difficult to externally validate the reliability of the reading results. Therefore, there is a possibility that doctors using the electrocardiogram reading service may not trust the reading results and may not use the electrocardiogram reading service.

### Disclosure

### Technical Problem

The present disclosure is not limited to diagnosing a disease as a result of electrocardiogram reading, but is intended to provide a method for providing additional information that can supplement diagnosis results, as in the case of an additional test or measures to be taken, and describe the basis for reading results.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of providing an electrocardiogram reading service. The method may include: generating first additional information about reading results included in a reading text by analyzing the reading text generated using a first machine learning model pre-trained to diagnose a disease based on an electrocardiogram; generating information related to the first machine learning model, used to generate the reading text, as second additional information; and providing at least one of the first additional information and the second additional information through a user interface.

Alternatively, generating first additional information about reading results included in a reading text by analyzing the reading text generated using a first machine learning model pre-trained to diagnose a disease based on an electrocardiogram may include: extracting at least one of the name of a disease and an electrocardiogram feature, included in the reading text, as a keyword; and generating first additional information, including at least one of a prescription, a test, and follow-up measures related to the extracted keyword, by using a previously constructed keyword database or a second machine learning model that is a pre-trained generative model.

Alternatively, the previously constructed keyword database may manage data in a data structure that includes key data corresponding to the name of each disease, sub-key data corresponding to at least one of the names of a prescription, a test, and follow-up measures associated with the name of the disease, and content data corresponding to the sub-key data as constituent elements.

Alternatively, generating first additional information, including at least one of a prescription, a test, and follow-up measures related to the extracted keyword, by using a previously constructed keyword database or a second machine learning model that is a pre-trained generative model may include: deriving key data matching the extracted keyword from the previously constructed keyword database; and generating the first additional information by extracting sub-key data and content data based on the derived key data.

Alternatively, the second machine learning model may be a model that has been pre-trained to generate information about a prescription, a test, and follow-up measures related to a keyword included in training data based on the training data including at least one of reading text data written or reviewed by medical staff and medical electronic record data.

Alternatively, the first machine learning model may be a model that has been pre-trained to receive an electrocardiogram and output a score indicating the possibility of the onset or progression of a disease.

Alternatively, the second additional information may include at least one of a cutoff value corresponding to a reading criterion for a score output from the first machine learning model, the prevalence of a disease analyzed by the first machine learning model, the sensitivity of the first machine learning model, the specificity of the first machine learning model, the training data of the first machine learning model, and the validation data of the first machine learning model.

Alternatively, the cutoff value or the prevalence may be determined based on information about the environment in which electrocardiogram data input to the first machine learning model was measured.

Alternatively, the sensitivity or the specificity may be determined based on the determined cutoff value.

Alternatively, providing at least one of the first additional information and the second additional information through a user interface may include visualizing at least one of the first additional information and the second additional information together with the reading text in response to a user request input through the user interface.

According to one embodiment of the present disclosure for achieving the above-described object, there is disclosed a computer program stored in a computer-readable storage medium. The computer program performs operations for providing an electrocardiogram reading service when executed on one or more processors. In this case, the operations may include operations of: generating first additional information about reading results included in a reading text by analyzing the reading text generated using a first machine learning model pre-trained to diagnose a disease based on an electrocardiogram; generating information related to the first machine learning model, used to generate the reading text, as second additional information; and providing at least one of the first additional information and the second additional information through a user interface.

According to one embodiment of the present disclosure for achieving the above-described object, there is disclosed a computing device for providing an electrocardiogram reading service. The computing device may include: a processor including at least one core; and memory including program codes executable on the processor. In this case, the processor generates first additional information about reading results included in a reading text by analyzing the reading text generated using a first machine learning model pre-trained to diagnose a disease based on an electrocardiogram, generates information related to the first machine learning model, used to generate the reading text, as second additional information, and provides at least one of the first additional information and the second additional information through a user interface.

### Advantageous Effects

The present disclosure is not limited to diagnosing a disease as a result of electrocardiogram reading, but provides additional information that can supplement diagnosis results, as in the case of an additional test or measures to be taken, and describe the basis for reading results, thereby increasing the reliability of the reading results and helping a customer accurately understand the reading results.

### Description of Drawings

FIG. 1 is a block diagram of a computing device according to one embodiment of the present disclosure;
FIG. 2 is a block diagram showing a process of providing an electrocardiogram reading service according to one embodiment of the present disclosure; and
FIG. 3 is a flowchart showing a method of providing an electrocardiogram reading service according to one embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

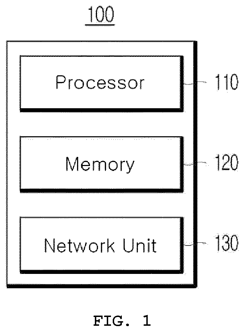

FIG. 1 is a block diagram of a computing device according to one embodiment of the present disclosure.

The computing device 100 according to the one embodiment of the present disclosure may be a hardware device or a part of a hardware device that performs the comprehensive processing and operation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a component unit including hardware and/or software for performing computing operation. For example, the processor 110 may read a computer program and perform data processing. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure. The processor 110 may generate first additional information about reading results included in a reading text by analyzing the reading text generated based on artificial intelligence. The processor 110 may analyze the text included in the reading text, and may derive information matching the analyzed text by using a previously constructed database or machine learning model. In this case, the first additional information may be understood as information that can help to diagnose a disease, such as a prescription, a test, or follow-up measures related to a disease derived from the reading results. For example, when the reading text generated based on artificial intelligence simply includes content indicating that a certain disease may be present, such as "atrial fibrillation is suspected," the processor 110 may extract the keyword "atrial fibrillation" by analyzing the text of the reading text. Furthermore, the processor 110 may extract information about an additional test and treatment related to "atrial fibrillation" by using a previously constructed database or machine learning model. The processor 110 may organize the extracted information into text and generate first additional information in the form of the information "If it is a first finding, a cardiologist's treatment is required. An additional 24-hour electrocardiogram test is required and antithrombotic medication is required. Aspirin is prescribed and a referral to a hospital having a cardiologist is recommended." In this case, the database from which the processor 110 extracts information may be a collection of data in which diseases and supplementary information such as prescriptions, tests, or follow-up measures related to the diseases are managed in an integrated manner. Moreover, the machine learning model may be a model that has been pre-trained to generate supplementary information such as a prescription, a test, or follow-up measures related to a keyword based on the keyword derived by analyzing the text of the reading text.

As described above, through the first additional information generated by the processor 110, a customer who requested an electrocardiogram reading may smoothly perform a diagnosis of a disease or additional measures without performing additional medical determination or information collection. In particular, there are many cases where non-specialists who use an electrocardiogram service often do not know what additional test or treatment has to be performed when a disease is suspected through the analysis of an electrocardiogram, so that the first additional information may provide practical help in diagnosing a disease and responding to it immediately.

The processor 110 may generate second additional information related to the artificial intelligence model used to analyze the reading text in order to provide not only the reading results but also the basis for the reading results through the service. When the reading text is generated, the processor 110 may identify the artificial intelligence model used to analyze the reading text. Furthermore, the processor 110 may generate second additional information corresponding to the basis for which the reading results were derived by analyzing the type of data used to develop the artificial intelligence model, the indicator related to the output of the artificial intelligence model, the processing method for the output of the artificial intelligence model, etc. In this case, the analysis of the artificial intelligence model performed to generate the second additional information may be performed based on a database in which information about the data used to develop or update the artificial intelligence model, information related to the data input to the artificial intelligence model for the reading, information related to the output of the artificial intelligence model, and/or the like are managed. For example, the processor 110 may analyze the specifications of the machine learning model used to generate the reading text, the data itself used for the reading or derived as the results of the reading, or information related to the data. More specifically, the processor 110 may generate at least one of the data used to develop the machine learning model used for the reading, the output accuracy of the model, information about a hospital that validated the output accuracy of the model, the sensitivity of the model, the specificity of the model, and statistical information, such as the prevalence of a disease analyzed by the model, as the second additional information.

As described above, through the second additional information generated by the processor 110, a customer who requested electrocardiogram reading may obtain not only reading results, but also information about the basis for which the reading results were derived, such as what data was used to construct the model used for the reading, what research results were used to validate them, and what the prevalence of a disease derived from the reading results is. Furthermore, the second additional information may include not only the value of the probability that the model determines that there is a disease by analyzing data, but also the reliability (certainty, or uncertainty) of the determination, so that the reliability of the reading results can be increased and the customer can be helped to accurately understand the meaning of the reading results.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize and manage data required for the processor 110 to perform operations, combinations of the data, and program codes executable by the processor 110. For example, the memory 120 may store the medical data received via the network unit 130 to be described later. The memory 120 may store program codes intended for storing rules for processing medical data, program codes intended for operating a neural network model to receive medical data and perform learning, program codes intended for operating a neural network model to receive medical data and perform inference according to the purpose of use of the computing device 100, and processed data generated as program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired/wireless communication with any system or client or the like. Furthermore, the network unit 130 may transmit data generated through the operation of the processor 110 through wired/wireless communication with any system or client or the like. For example, the network unit 130 may receive medical data through communication with a database within a hospital environment, a cloud server configured to perform tasks such as the standardization of medical data, a client such as a smart watch, a medical computing device, or the like. The network unit 130 may transmit the output data of the neural network model, intermediate data and processed data obtained from the operation process of the processor 110, and/or the like through communication with the above-described database, server, client or computing device.

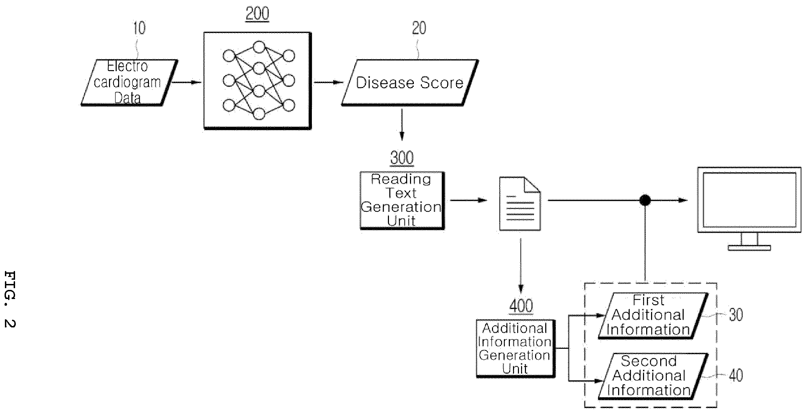

FIG. 2 is a block diagram showing a process of providing an electrocardiogram reading service according to one embodiment of the present disclosure.

The computing device 100 according to one embodiment of the present disclosure may calculate a disease score 20 indicating the possibility of the onset or progression of a disease by inputting electrocardiogram data 10 to a first machine learning model 200. In this case, the disease score 20 may be represented by a numerical value in the range of 0 to 100 points. As the disease score 20 becomes closer to 0, the possibility of the onset of the disease decreases and a healthier state is obtained, and in contrast, as it becomes closer to 100, the possibility of the onset of the disease increases and a more dangerous state having higher disease severity is obtained.

Meanwhile, the first machine learning model 200 may be a model trained based on training data including electrocardiogram data and labels each indicating the possibility of the onset or progression of a disease. For example, the first machine learning model 200 may receive electrocardiogram data included in training data and extract electrocardiogram features. In this case, the electrocardiogram features may be understood as features identified from the waveforms of the electrocardiogram signal, such as the P wave, the QRS complex, and the T wave. The first machine learning model 200 may calculate each disease score based on the extracted electrocardiogram features. Furthermore, the first machine learning model 200 may update the neural network parameters by comparing the calculated disease scores with the labels. In this case, the comparison between the disease scores and the labels may be performed based on a loss function. When the error between the disease scores and the labels is calculated through the loss function, the first machine learning model 200 may repeatedly perform a process of updating the neural network parameters until the error satisfies a minimum criterion. The first machine learning model 200 may be trained through self-supervised learning, including a neural network including an attention layer, other than supervised learning such as that of the example described above.

The computing device 100 may determine whether a disease has occurred based on the disease score 20. The computing device 100 may determine whether a subject from whom the electrocardiogram data 10 was measured has developed a disease by comparing the disease score 20 with a cutoff value. For example, when the disease score 20 is equal to or higher than the cutoff value, the computing device 100 may determine that the subject from whom the electrocardiogram data 10 was measured has developed a disease. When the disease score 20 is lower than the cutoff value, the computing device 100 may determine that the subject from whom the electrocardiogram data 10 was measured has not developed a disease. In this case, the cutoff value may be understood as a value that dynamically changes depending on the environment in which the electrocardiogram data 10 is measured. When the measurement environment is a health checkup center having a low level of emergency out of medical environments, the cutoff value may increase, and in contrast, when the measurement environment is an intensive care unit having a high level of emergency out of medical environments, the cutoff value may decrease.

The computing device 100 may generate a reading text by organizing reading results into a text format through a reading text generation unit 300 implemented through the processor 110. For example, the reading text may be in the form in which reading information is listed as text, as in the example "CONSIDER RIGHT VENTRICULAR HYPERTROPHY ... large R or R' V1/V2," "SUPRAVENTRICULAR TACHYCARDIA, RATE 231 ... V-rate>(220-age) or 150." In this case, the reading information listed in the reading text may include the name of a disease, electrocardiogram features of the corresponding disease, etc.

The computing device 100 may extract a keyword from text-type reading results included in a reading text through an additional information generation unit 400 implemented through the processor 110. The computing device 100 may generate first additional information 30 including at least one of the prescription, test, and follow-up measures of a disease related to the keyword extracted through the additional information generation unit 400. In this case, the first additional information 30 may be generated through a previously constructed keyword database or a second machine learning model that is a pre-trained generative model.

For example, the additional information generation unit 400 may extract at least one of the name of a disease and an electrocardiogram feature as a keyword from the text-type reading information included in the reading text. The additional information generation unit 400 may generate the first additional information 30 by searching the keyword database in which information about at least one of the prescription, test, and follow-up measures of the disease matching the extracted keyword is stored. In this case, the keyword database may manage data in a data structure that includes key data corresponding to the name of each disease, sub-key data corresponding to at least one of the names of a prescription, a test, and follow-up measures associated with the name of the disease, and content data corresponding to the sub-key data as constituent elements. The additional information generation unit 400 may search for key data matching a keyword corresponding to at least one of the name of a disease and an electrocardiogram feature in the keyword database in which data is structured in the form of [key data - sub-key data - content data]. Furthermore, the additional information generation unit 400 may generate the first additional information 30 by extracting sub-key data and content data associated with key data from the keyword database.

In addition, the additional information generation unit 400 may generate the first additional information 30 through a second machine learning model that generates information about at least one of a prescription, a test, and follow-up measures related to a keyword extracted from a reading text. The additional information generation unit 400 may generate information about at least one of a prescription, a test, and follow-up measures related to a keyword as data in a text form by inputting at least one of the name of a disease and an electrocardiogram feature, extracted from a reading text, to the second machine learning model. In this case, the type of data input to the second machine learning model may be a keyword itself extracted from the reading text, or may be a sentence including the keyword. Assuming that the name of a disease extracted from the reading text is atrial fibrillation, the additional information generation unit 400 may generate information about at least one of a prescription, a test, and follow-up measures related to atrial fibrillation, such as the information "If atrial fibrillation is a first finding, a cardiologist's treatment is required. An additional 24-hour electrocardiogram test is required and antithrombotic medication is required. Aspirin is prescribed and a referral to a hospital having a cardiologist is recommended" as the first additional information 30 by inputting the word "atrial fibrillation" or the sentence "atrial fibrillation is suspected" to the second machine learning model.

Meanwhile, the second machine learning model may be a model that has been pre-trained to generate information about a prescription, a test, and follow-up measures related to a keyword included in training data based on the training data including at least one of reading text data written or reviewed by medical staff and medical electronic record data. For example, the second machine learning model may include a neural network capable of processing sequential data, such as a recurrent neural network (RNN), a long short term memory (LSTM) neural network, or a transformer. Furthermore, the second machine learning model may be trained based on supervised learning through labels included in training data, or may be trained through self-supervised learning.

The computing device 100 may generate information related to the first machine learning model 200 used to generate a reading text as second additional information 40 through the additional information generation unit 400 implemented through the processor 110. In this case, the second additional information 40 may include at least one of a cutoff value corresponding to a reading criterion for a score output from the first machine learning model 200, the prevalence of a disease analyzed by the first machine learning model 200, the sensitivity of the first machine learning model 200, the specificity of the first machine learning model 200, the training data of the first machine learning model 200, and the validation data of the first machine learning model 200. The cutoff value or prevalence may be determined based on information about the environment in which the electrocardiogram data 10 input to the first machine learning model 200 was measured. Furthermore, the sensitivity or specificity may be determined based on the determined cutoff value.

For example, when the computing device 100 obtains the electrocardiogram data 10, it may obtain information about the environment in which the electrocardiogram data 10 was measured. The information about the environment may include information about the place where the electrocardiogram data 10 was measured, such as a health checkup center, a general ward, or an intensive care unit. The computing device 100 may determine a cutoff value, used to determine whether a disease has developed based on the disease score 20, by using the information about the environment. When the electrocardiogram data 10 is measured in a routine medical environment such as a home or a health checkup center, the computing device 100 may determine the cutoff value to be a relatively high value. In contrast, when the electrocardiogram data 10 is measured in an unusual medical environment such as an intensive care unit, the computing device 100 may determine the cutoff value to be a relatively low value. The computing device 100 may flexibly determine the cutoff value in accordance with the information about the environment in this manner. The computing device 100 may determine the prevalence of the environment identified from the information about the environment by searching a database that stores information about the relationships between environments and prevalence rates. The computing device 100 may calculate the sensitivity and specificity of the first machine learning model 200 according to the cutoff value determined based on the information about the environment. Furthermore, the computing device 100 may collect information about data used for the training or validation of the first machine learning model 200 by searching a database that stores information about data used for the development or update of the first machine learning model 200. The computing device 100 may generate second additional information 40 by compiling the information about the cutoff value, the prevalence, the sensitivity, the specificity, and the data used for the training or the validation.

The computing device 100 may generate a user interface for providing a reading text. Furthermore, the computing device 100 may additionally provide at least one of the first additional information 30 and the second additional information 40 through the user interface for providing a reading text. For example, when the reading of the electrocardiogram data 10 is completed, the computing device 100 may provide a reading text to the terminal of the customer, who requested electrocardiogram reading, through the user interface and visualize it first. In this case, when a user request for the viewing of the additional information is input through the user interface, the computing device 100 may provide at least one of the first additional information 30 and the second additional information 40 to the terminal of the customer through the user interface and visualize it together with the reading text. In this case, at least one of the first additional information 30 and the second additional information 40 may be visualized while being overlaid on the reading text, or may be visualized in an area separate from the reading text. The computing device 100 may visualize the reading text and at least one of the first additional information 30 and the second additional information 40 simultaneously by providing them to the terminal of the customer, who requested electrocardiogram reading, through the user interface.

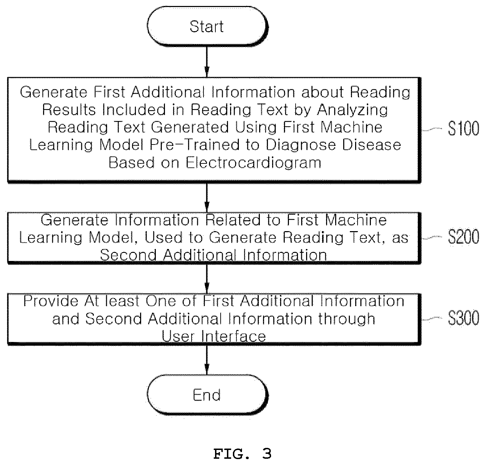

FIG. 3 is a flowchart showing a method of providing an electrocardiogram reading service according to one embodiment of the present disclosure.

Referring to FIG. 3, the computing device 100 according to one embodiment of the present disclosure may generate first additional information about reading results included in a reading text by analyzing the reading text generated using the first machine learning model pre-learned to diagnose a disease based on an electrocardiogram in step S100. The computing device 100 may extract at least one of the name of a disease and an electrocardiogram feature, included in a reading text, as a keyword. The computing device 100 may generate first additional information, including at least one of a prescription, a test, and follow-up measures related to the extracted keyword, by using the previously constructed keyword database or the second machine learning model that is a pre-trained generative model. More specifically, the computing device 100 may derive key data matching the extracted keyword from the previously constructed keyword database. The computing device 100 may generate first additional information by extracting sub-key data and content data based on the derived key data. In this case, the extraction of the first additional information may be performed by a user request through the user interface. When the user requests the viewing of the first additional information through the user interface, the computing device 100 may generate the first additional information by checking the key data, the sub-key data, and the content data. However, since a user request for the extraction of the first additional information is not necessarily required, the computing device 100 may generate the first additional information immediately without a user request when the reading text is generated. The computing device 100 may also generate the first additional information in the form of text including at least one of a prescription, a test, and follow-up measures related to a keyword by inputting the keyword to the second machine learning model. In this case, the second machine learning model may be a model that has been pre-trained to generate information about a prescription, a test, and follow-up measures related to a keyword included in training data based on the training data including at least one of reading text data written or reviewed by medical staff and medical electronic record data.

The computing device 100 may generate information related to the first machine learning model, used to generate the reading text, as second additional information in step S200. Since the reading of the electrocardiogram is performed using an artificial intelligence model such as the first machine learning model, it is important to check the basis for which reading based on the output of the first machine learning model was performed for the reliability of the reading. Accordingly, the computing device 100 may generate information about the data used for the development and update of the first machine learning model and the evaluation of the performance of the first machine learning model according to various indicators as second additional information. For example, the second additional information may include at least one of a cutoff value corresponding to a reading criterion for a score output from the first machine learning model, the prevalence of a disease analyzed by the first machine learning model, the sensitivity of the first machine learning model, the specificity of the first machine learning model, information about the training data of the first machine learning model, or information about the validation data of the first machine learning model. In this case, the cutoff value or the prevalence may be determined based on information about the environment in which the electrocardiogram data input to the first machine learning model was measured. Furthermore, the sensitivity and the specificity may be determined by mathematical computation based on the determined cutoff value.

The computing device 100 may provide at least one of the first additional information and the second additional information through the user interface in step S300. The computing device 100 may visualize at least one of the first additional information and the second additional information together with the reading text and provide them to the terminal of the customer, who requested the reading of the electrocardiogram, through the user interface. In this case, at least one of the first additional information and the second additional information together with the reading text may be visualized simultaneously and exposed on the display of the terminal, or they may be selectively visualized and exposed on the display of the terminal according to a user request.

For example, the computing device 100 may include both the first additional information and the second additional information in the reading text, and may visualize the resulting reading text in the form of text, as follows:

"As a result of electrocardiogram reading requested by Mr. OO, the possibility of myocardial infarction is 3.7 points. The prevalence of myocardial infarction at this health checkup center is 0.5%, and our recommended cutoff value is 40 points. When the cutoff value of 40 points is applied, the accuracy of the artificial intelligence is 80%, the sensitivity thereof is 70%, and the specificity thereof is 65%. This artificial intelligence model was trained based on the data measured at this health checkup center for OO adults during the period from 2018 to 2022 and was validated based on the electrocardiograms measured at Hospital A during the period from 2013 to 2015, and the accuracy thereof was OO%."

In addition, the computing device 100 may visualize the reading text in a text form alone without the first additional information and the second additional information. In this case, when a customer performs a mouse-over action of hovering a mouse cursor on a keyword such as the name of a disease or an electrocardiogram feature in the text included in the reading text, at least one of the first additional information and the second additional information related to the selected keyword may be visualized in a text form through an action such as a pop-up.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of providing an electrocardiogram reading service, the method being performed by a computing device including at least one processor, the method comprising:
generating first additional information about reading results included in a reading text by analyzing the reading text generated using a first machine learning model pre-trained to diagnose a disease based on an electrocardiogram;
generating information related to the first machine learning model, used to generate the reading text, as second additional information; and
providing at least one of the first additional information and the second additional information through a user interface.

2. The method of claim 1, wherein generating first additional information about reading results included in a reading text by analyzing the reading text generated using a first machine learning model pre-trained to diagnose a disease based on an electrocardiogram comprises:
extracting at least one of a name of a disease and an electrocardiogram feature, included in the reading text, as a keyword; and
generating first additional information, including at least one of a prescription, a test, and follow-up measures related to the extracted keyword, by using a previously constructed keyword database or a second machine learning model that is a pre-trained generative model.

3. The method of claim 2, wherein the previously constructed keyword database manages data in a data structure that includes key data corresponding to a name of each disease, sub-key data corresponding to at least one of names of a prescription, a test, and follow-up measures associated with the name of the disease, and content data corresponding to the sub-key data as constituent elements.

4. The method of claim 3, wherein generating first additional information, including at least one of a prescription, a test, and follow-up measures related to the extracted keyword, by using a previously constructed keyword database or a second machine learning model that is a pre-trained generative model comprises:
deriving key data matching the extracted keyword from the previously constructed keyword database; and
generating the first additional information by extracting sub-key data and content data based on the derived key data.

5. The method of claim 2, wherein the second machine learning model is a model that has been pre-trained to generate information about a prescription, a test, and follow-up measures related to a keyword included in training data based on the training data including at least one of reading text data written or reviewed by medical staff and medical electronic record data.

6. The method of claim 1, wherein the first machine learning model is a model that has been pre-trained to receive an electrocardiogram and output a score indicating a possibility of onset or progression of a disease.

7. The method of claim 6, wherein the second additional information comprises at least one of a cutoff value corresponding to a reading criterion for a score output from the first machine learning model, a prevalence of a disease analyzed by the first machine learning model, a sensitivity of the first machine learning model, a specificity of the first machine learning model, training data of the first machine learning model, and validation data of the first machine learning model.

8. The method of claim 7, wherein the cutoff value or the prevalence is determined based on information about an environment in which electrocardiogram data input to the first machine learning model was measured.

9. The method of claim 7, wherein the sensitivity or the specificity is determined based on the determined cutoff value.

10. The method of claim 1, wherein providing at least one of the first additional information and the second additional information through a user interface comprises visualizing at least one of the first additional information and the second additional information together with the reading text in response to a user request input through the user interface.

11. A computer program stored in a computer-readable storage medium, the computer program performing operations for providing an electrocardiogram reading service when executed on one or more processors, wherein the operations comprise operations of:
generating first additional information about reading results included in a reading text by analyzing the reading text generated using a first machine learning model pre-trained to diagnose a disease based on an electrocardiogram;
generating information related to the first machine learning model, used to generate the reading text, as second additional information; and
providing at least one of the first additional information and the second additional information through a user interface.

12. A computing device for providing an electrocardiogram reading service, the computing device comprising:
a processor including at least one core; and
memory including program codes executable on the processor;
wherein the processor:
generates first additional information about reading results included in a reading text by analyzing the reading text generated using a first machine learning model pre-trained to diagnose a disease based on an electrocardiogram;
generates information related to the first machine learning model, used to generate the reading text, as second additional information; and
provides at least one of the first additional information and the second additional information through a user interface.
